(19) Europäisches Patentamt
European Patent Office
Office européen des brevets

(11) Publication number: 0 182 317 A2

# (12) EUROPEAN PATENT APPLICATION

(21) Application number: 85114557.3

(22) Date of filing: 16.11.85

(51) Int. Cl.[4]: A 61 K 31/095, A 61 K 33/04

(30) Priority: 20.11.84 JP 243173/84

(43) Date of publication of application: 28.05.86 Bulletin 86/22

(84) Designated Contracting States: DE FR GB IT

(71) Applicant: NIPPON KAYAKU KABUSHIKI KAISHA, 11-2, Fujimi 1-chome Chiyoda-ku, Tokyo 102 (JP)

(72) Inventor: Suzuki, Masanobu, 1090, Kamiochiai, Yono-shi Saitama-ken (JP)
Inventor: Izawa, Takao, 1-10-20, Buzo, Urawa-shi Saitama-ken (JP)
Inventor: Ekimoto, Hisao, 2-11-1-803, Shimo, Kita-ku Tokyo (JP)
Inventor: Takahashi, Katsutoshi, 3-10-8-512, Kamiya, Kita-ku Tokyo (JP)
Inventor: Nakatani, Tokuji, 4-41-4, Sakae Ina-machi, Kitaadachi-gun Saitama-ken (JP)
Inventor: Fujii, Akio, 4-8-13, Oofuna, Kamakura-shi Kanagawa-ken (JP)

(74) Representative: Türk, Dietmar, Dr. rer. nat. et al, Patentanwälte Türk & Gille Brucknerstrasse 20, D-4000 Düsseldorf 13 (DE)

## (54) Antineoplastic agent.

(57) The present invention relates to an antineoplastic agent containing a selenium compound of the general formula:

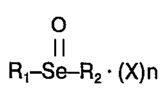

wherein $R_1$ represents a halogen atom, a phenyl group, a lower alkoxy group or a halogen-substituted lower alkoxy group, $R_2$ represents a halogen atom, a hydroxy group, a lower alkoxy group or a halogen-substituted lower alkoxy group or $R_1$ and $R_2$ together form a group of the formula:

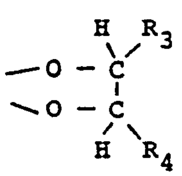

or

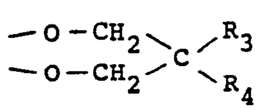

(in which $R_3$ and $R_4$ each represent a hydrogen atom or a lower alkyl group), X represents pyridine, quinoline, isoquinoline or dioxane and n represents an integer of 0 to 2,

$$Se(Hal)_4 \cdot (Y)m$$

wherein Hal represents a halogen atom, Y represents pyridine and m represents 0 or 2.

EP 0 182 317 A2

# ANTINEOPLASTIC AGENT

## Background of the Invention:

The methods of synthesis and some physicochemical properties of the selenium compounds of the present invention have already been known. However, medical properties such as an antineoplastic activity of them have not been reported yet. Malignant tumors have various properties and become resistant to antineoplastic agents after repeated use thereof. The development of new antineoplastic agents is, therefore, demanded.

## Summary of the Invention:

After intensive investigations of compounds having an antineoplastic effect, the inventors have found that selenium compounds of the following general formula have an antineoplastic effect:

$$R_1 - \overset{O}{\overset{\|}{Se}} - R_2 \cdot (X)_n \qquad (I)$$

wherein $R_1$ represents a halogen atom, a phenyl group, a lower alkoxy group or a halogen-substituted lower alkoxy group, $R_2$ represents a halogen atom, a hydroxy group, a lower alkoxy group or a halogen-substituted lower alkoxy group

or $R_1$ and $R_2$ together form a group of the formula:

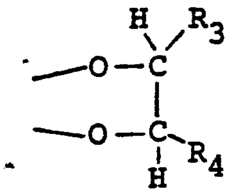

or

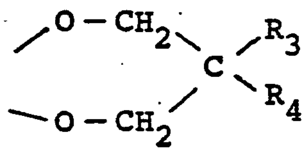

(in which $R_3$ and $R_4$ each represent a hydrogen atom or a lower alkyl group), X represents pyridine, quinoline, isoquinoline or dioxane and n represents an integer of 0 to 2,

or

$$Se(Hal)_4 \cdot (Y)_m \qquad (II)$$

wherein Hal represents a halogen atom, Y represents pyridine and m represents 0 or 2.

The present invention has been completed on the basis of this finding.

Detailed Description of the Invention:

When $R_1$ and $R_2$ in the above general formula (I) are each a halogen atom, they may be fluorine, chlorine, bromine or iodine. Among them, chlorine or bromine is preferred. Examples of the lower alkoxy groups as $R_1$ and $R_2$ include methoxy, ethoxy, propoxy, isopropoxy, butoxy, sec-butoxy, t-butoxy and pentyloxy groups. Among them, ethoxy and propoxy groups are preferred. The halogen-substituted lower alkoxy

groups are preferably chlorinated ones having 2 or 3 carbon atoms, such as 2-chloroethoxy and 2-chloropropoxy groups.

When $R_1$ and $R_2$ are bonded together to form the above-mentioned group, the lower alkyl group as $R_3$ or $R_4$ is, for example, a methyl group. Examples of the groups of the formula: 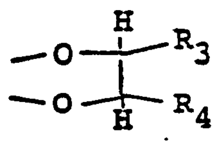 formed by bonding $R_1$ and $R_2$ together include 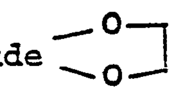 , 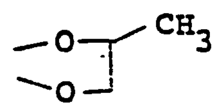 , and 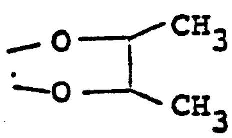 and examples of the groups of the formula: 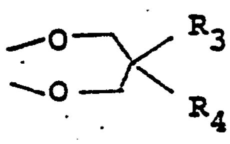 include 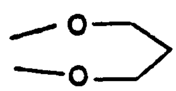 , 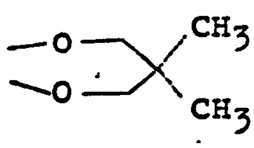 .

In a preferred combination of $R_1$, $R_2$, X and n in the general formula (I), $R_1$ and $R_2$ are each a chlorine atom or an ethoxy group or $R_1$ and $R_2$ are bonded together to form 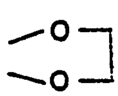 wherein X is pyridine and n is 0 or 2.

In a preferred combination of Hal, Y and m in the general formula (II), Hal is a chlorine or bromine

atom, Y is pyridine and m is 0 or 2.

Typical examples of the compounds of the general formula (I) or (II) are as follows, which will be referred to as shown in the parentheses hereinafter:

| | | |
|---|---|---|
| 1. | phenylseleninic acid, | |
| 2. | ethyl phenylseleninate, | |
| 3. | selenium oxychloride, | (SC) |
| 4. | selenium oxychloride·pyridine complex | (SCPY) |
| 5. | selenium oxychloride·quinoline complex | (SCQn) |
| 6. | selenium oxychloride·isoquinoline complex | (SCIQn) |
| 7. | selenium oxychloride·dioxane complex | (SCDX) |
| 8. | selenium oxybromide·dioxane complex | (SBDX) |
| 9. | diethyl selenite | (SOEt) |
| 10. | di(n-propyl) selenite | (SOPr) |
| 11. | diisopropyl selenite | (SOIp) |
| 12. | di(2-chloroethyl) selenite | (SOCE) |
| 13. | di(2-chloropropyl) selenite | (SOCP) |
| 14. | 1,2-ethanediol selenite | (SOEC) |
| 15. | 1,2-propanediol selenite | (SOMEC) |
| 16. | 2,3-butanediol selenite | (SODEC) |
| 17. | 1,3-propanediol selenite | (SOPC) |
| 18. | 2,2-dimethyl-1,3-propanediol selenite | (SOMPC) |
| 19. | selenium tetrachloride | ($C_4S$) |
| 20. | selenium tetrabromide | ($B_4S$) |
| 21. | selenium tetrachloride·pyridine complex | ($C_4SPY$) |

Among these compounds, those of Nos. 4, 5, 9, 14, 15, 16, 19, 20 and 21 are preferred.

The phenylseleninic acid may be used in the form of its salt with sodium, potassium, ammonium or the like. A process for producing these compounds and ethyl phenylseleninate is described by G. Ayrey et. al. in J. Chem. Soc. 1962, 2089.

A process for producing a complex of selenium oxychloride or selenium tetrachloride with pyridine is reported by B. Edgington & J.B. Firth in J. Soc. Chem. Ind., 55, 192T (1936). The molar ratio of selenium oxychloride to pyridine in this complex is either 1:1 or 1:2. Both are included in the scope of the present invention.

A process for producing a complex of selenium oxychloride with quinoline or isoquinoline is reported by J. Jackson & G.B.L. Smith in J. Am. Chem. Soc. 62, 544 (1940).

The molar ratio of selenium oxychloride to quinoline or isoquinoline in this complex is either 1:1 or 1:2. Both are included in the scope of the present invention.

A process for producing a complex of selenium oxychloride or selenium oxybromide with dioxane, di-(2-chloroethyl) selenite and di(2-chloropropyl) selenite

is reported by N. N. Yarovenko et al. in Zh. Obshch. Khim. 31, 4006 (1961).

A process for producing diethyl selenite, di-(n-propyl) selenite and diisopropyl selenite is reported by F. Ando et al. in Bull, Chem. Soc. Jpn. 52, 807 (1979).

A process for producing 1,2-ethanediol selenite and 2,2-dimethyl-1,3-propanediol selenite is reporated by D. B. Denney et al. in J. Am. Chem. Soc. 103, 2340 (1981).

A process for producing 1,2-propanediol selenite, 2,3-butanediol selenite and 1,3-propanediol selenite is reporated by R. C. Mehrotra & S. N. Mathur in J. Indian. Chem. Soc. 42, 814 (1965).

A process for producing selenium oxychloride is reporated by Drago & Whitten in Inorg. Chem. 5, 677 (1966).

A process for producing selenium tetrachloride is reported by Lamb in Am. Chem. J. 30, 209 (1903).

A process for producing selenium tetrabromide is reported by Pogg in Ann. 128, 327 (1866).

The antineoplastic agent of the present invention can be formulated and administered by various known methods. The methods of administration include, for example, injection, oral and rectal

administration. The forms of the preparation include injection, powder, granules, tablets and suppositories.

In the formulation, medical additives such as carriers, excipients, stabilizers, antiseptics, soothing agents and emulsifiers may be used if necessary so far as they do not exert an adverse effect on the selenium compound. Examples of the excipients and stabilizers include mannitol, lactose, maltose, dextran, starch and polyethylene glycol.

Examples of the antiseptics include benzalkonium chloride, benzyl alcohol and parabens.

Examples of the soothing agents include benzyl alcohol, inositol and glucose.

Examples of the emulsifiers include polysorbates and fatty acid esters.

Examples of the base materials for the suppositories include fatty acid triglycerides, polyethylene glycol and polyvinyl alcohol.

The amount of the selenium compound in the antineoplastic agent which is variable over a wide range depending on the form of the antineoplastic agent is generally 0.01 to 100 wt. %, preferably 0.1 to 70 wt. % and the balance comprises ordinary medicinal carrier and other additives.

An effective dose of the antineoplastic agent

of the present invention in the above-mentioned form is applied to the patients. The dosages determined on the basis of the results of basic experiments of pharmacological effects are 0.01 to 10 mg/kg/day, preferably 0.1 to 2 mg/kg/day in the oral administration, 0.01 to 5 mg/kg/day, preferably 0.1 to 1 mg/kg/day for the injection and 0.01 to 10 mg/kg/day, preferably 0.1 to 2 mg/kg/day for the suppositories.

The antineoplastic effect of the selenium compounds of the present invention will be shown by the following experiments:

Experiment 1

Effect of inhibiting the proliferation of incubated Hela S3 cells:

[Sample]

Selenium oxychloride·pyridine (1:1) complex was used as the sample.

[Method of experiment]

Hela S3 cells were placed on a culture medium (NEM containing 10 % of calf serum in a plastic dish. After 2 days, an organoselenium compound was added thereto. After containing the culture for additional 3 days, the number of the cells was counted. The proliferation inhibition rate was calculated according to the following formula:

Inhibition rate (%) = 100 x (B-A)/(B-C)

in which A is the final number of the cells counted on the third day after addition of the test sample, B is the final number of the cells in a sample-free control and C is the number of the cells counted immediately after the addition of the test sample.

The value of $IC_{50}$ (50 % inhibitory concentration) was determined from a graph showing the relationship between the concentration of the sample and the inhibition rate.

[Results of experiment]

The results were as follows:

| Compound | $IC_{50}$ (µg/ml) |
|---|---|
| Selenium oxychloride·pyridine (1:1) complex | 0.59 |

Experiment 2

Antineoplastic effect on Ehrlich carcinoma:

Compounds shown in Tables 1 and 2 were used as the samples:

Table 1 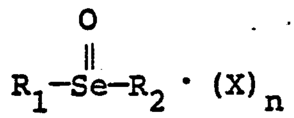

| No: | Abbreviated name | $R_1$ | $R_2$ | n | X |
|---|---|---|---|---|---|
| 1 | — | 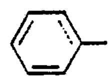 | -OH | 0 | — |
| 2 | — | 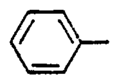 | $-OC_2H_5$ | 0 | — |
| 3 | SC | Cl | Cl | 0 | — |
| 4. | SCPY | Cl | Cl | 2 | 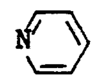 |
| 5 | $SCQ_n$ | Cl | Cl | 2 | 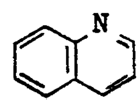 |
| 6 | $SCIQ_n$ | Cl | Cl | 2 | 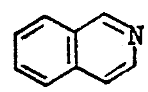 |
| 7 | SCDX | Cl | Cl | 1 | 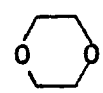 |
| 8 | SBDX | Br | Br | 1 | 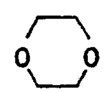 |
| 9 | SOET | $-OC_2H_5$ | $-OC_2H_5$ | 0 | — |
| 10 | SOPr | $-O-CH_2CH_2CH_3$ | $-OCH_2CH_2CH_3$ | 0 | — |
| 11 | SOIP | 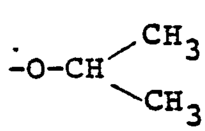 | 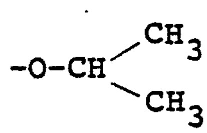 | 0 | — |

| | | | | | |
|---|---|---|---|---|---|
| 12 | SOCE | $-OCH_2CH_2Cl$ | $-OCH_2CH_2Cl$ | 0 | — |
| 13 | SOCP | $-OCH_2CHClCH_3$ | $-OCH_2CHClCH_3$ | 0 | — |
| 14 | SOEC | 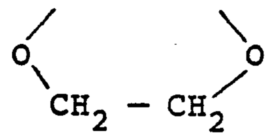 | | 0 | — |
| 15 | SOMEC | 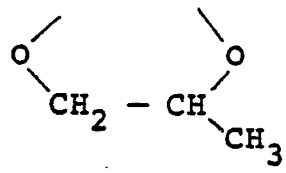 | | 0 | — |
| 16 | SODEC | 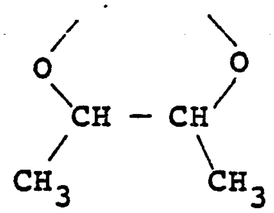 | | 0 | — |
| 17 | SOPC | 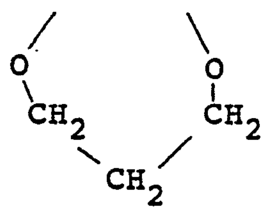 | | 0 | — |
| 18 | SOMPC | 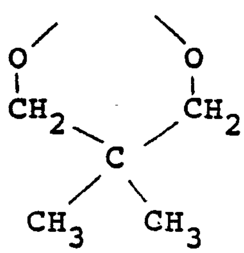 | | 0 | — |

Table 2. $Se(Hal)_4 \cdot (Y)_m$

| No. | Abbreviated name | Hal | m | Y |
|---|---|---|---|---|
| 19 | $C_4S$ | Cl | 0 | — |
| 20 | $B_4S$ | Br | 0 | — |
| 21 | $C_4SPY$ | Cl | 2 | 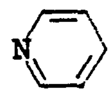 |

[Method of experiment]

About $10^6$/0.2 ml of Ehrlich carcinoma cells were placed in an abdomen of each male ICR mouse of 5 weeks old. On the next day the administration of the test sample in the abdomen was started. The test sample was given once a day for 7 days. For an untreated group, a physiological saline solution was given in the same manner as above. After the administration, the mice were observed for 30 days. The T/C percent (T/C x 100) was determined from the average survival periods (days) of the treated group and control group. Further, 50 % lethal dose ($LD_{50}$) was determined from the number of the mice which died of toxicity of the test samples administered.

The results are shown in Table 3.

Table 3

| Compound No. | Observation period (days) | Antineoplastic effect (dose:mg/kg) | | | | | | | | | | | | LD50 (mg/kg/day x 7) |
|---|---|---|---|---|---|---|---|---|---|---|---|---|---|---|
| | | 0.5 | 0.75 | 1.0 | 1.5 | 2.0 | 3.0 | 4.0 | 6.0 | 8.0 | 16.0 | 32.0 | 64.0 | |
| 1 | 30 | | | | | 122.6 | | 128.6 | | 141.4 | 148.9 | | | > 16 |
| | 60 | | | | | 123 | | 129 | | 141 | 149 | | | |
| 2 | 30 | | | | | | | 117.7 | | 133.8 | 150.0 | 87.9 | | 16 - 32 |
| | 60 | | | | | | | 118 | | 134 | 193 | 88 | | |
| 3 | 60 | | 141 | | 222 | | 168 | | 105 | | | | | 6 |
| 4 | 30 | | | | | 178.6 | | 188.1 | | 200.8 | 77.0 | | | 8 - 16 |
| | 60 | | | | | 218 | | 270 | | 297 | 95 | | | |
| 5 | 30 | | | | | 131.4 | | 156.0 | | 181.8 | 174.2 | | | > 16 |
| | 60 | | | | | 131 | | 199 | | 269 | 235 | | | |
| 6 | 30 | | | | | 158.5 | | 164.8 | | 162.9 | 77.4 | | | 8 - 16 |
| | 60 | | | | | 211 | | 206 | | 187 | 77 | | | |
| 7 | 60 | | | | | 211 | | 225 | | 194 | 40 | | | 8 - 16 |
| 8 | 60 | | | | | 182 | | 226 | | 228 | 78 | | | 8 - 16 |

(cont'd)

| Compound No. | Observation period (days) | Antineoplastic effect (dose: mg/kg) 0.5 | 0.75 | 1.0 | 1.5 | 2.0 | 3.0 | 4.0 | 6.0 | 8.0 | 16.0 | 32.0 | 64.0 | LD50 (mg/kg/day x7) |
|---|---|---|---|---|---|---|---|---|---|---|---|---|---|---|
| 9 | 60 | | | | | 246 | | 203 | | 201 | 14 | | | 4 – 8 |
| 10 | 60 | | | | | 175 | | 170 | | 102 | 14 | | | 8 |
| 11 | 60 | | | | | 204 | | 236 | | 126 | 14 | | | 4 – 8 |
| 12 | 60 | | | | | 197 | | 225 | | 194 | 65 | | | 8 |
| 13 | 60 | | | | | 190 | | 234 | | 219 | 71 | | | 8 – 16 |
| 14 | 60 | | | | | 193 | | 266 | | 108 | 34 | | | 4 – 8 |
| 15 | 60 | | | 227 | | 258 | | 204 | | 105 | | | | 8 |
| 16 | 60 | | | 185 | | 216 | | 261 | | 174 | | | | >8 |
| 17 | 60 | | | 174 | | 221 | | 197 | | 83 | | | | 4 – 8 |
| 18 | 60 | | | 164 | | 193 | | 194 | | 194 | | | | > 8 |
| 19 | 60 | | | 167 | | 179 | | 249 | | 183 | | | | >16 |
| 20 | 60 | | | | | 196 | | 272 | | 183 | 56 | | | 8 – 16 |
| 21 | 60 | | | | | 155 | | 217 | | 246 | 101 | | | 8 – 16 |

It is apparent from Table 3 that all the test compounds had a remarkable life-prolonging effect.

Experiment 3

Antimutagenic properties:

[Method of experiment]

The experiment was carried out by Ames method using Salmonella typhimurium TA-98 (hereinafter referred to as TA-98) as the test microorganism and benzo[α]pyrene (hereinafter referred to as B(α)P) as the mutagen.

0.1 ml of a solution containing 5 μg of B(α)P and a suitable amount of a compound of the present invention was added to 0.5 ml of S-9 mix and 0.1 ml of a TA-98 suspension. The mixture was incubated at 37°C for 20 min. 2 ml of soft agar was added thereto, mixed well and then spread evenly on a glucose minimum agar medium plate. After the incubation at 37°C for 48 h, the number of colonies formed by the back mutation was counted.

The number of the colonies formed when neither B(α)P nor the compound of the present invention was used was employed as the background.

The antimutagenic properties was shown as the rate of inhibition of the mutagenic effect according to the following formula:

inhibition rate (%)

$$= \left[ 1 - \left\{ \frac{\begin{pmatrix}\text{number of colonies formed} \\ \text{when the compound of the} \\ \text{invention was used}\end{pmatrix} - \begin{pmatrix}\text{back-} \\ \text{ground}\end{pmatrix}}{\begin{pmatrix}\text{number of colonies formed} \\ \text{when the compound of the} \\ \text{invention was not used}\end{pmatrix} - \begin{pmatrix}\text{back-} \\ \text{ground}\end{pmatrix}} \right\} \right] \times 100$$

The results are shown in Table 4.

Table 4

| Compound No. | | back-ground | dose (µg/plate) 0 | 0.5 | 1 | 5 | 10 | 50 | 100 | 500 |
|---|---|---|---|---|---|---|---|---|---|---|
| 4 | Number of colonies (inhibition rate; %) | 28<br>— | 272<br>(0) | 213<br>(23.0) | 192<br>(32.8) | 180<br>(37.7) | 144<br>(52.5) | 93<br>(73.4) | 87<br>(75.8) | 41<br>(94.7) |
| 14 | Number of colonies (inhibition rate ;%) | 25<br>— | 293<br>(0) | 231<br>(23.1) | 235<br>(21.6) | 156<br>(51.1) | 145<br>(55.2) | 76<br>(81.0) | 62<br>(86.2) | 32<br>(97.4) |
| 3 | Number of colonies (inhibition rate %) | 32<br>— | 286<br>(0) | 205<br>(30.3) | 230<br>(22.0) | 154<br>(52.0) | 132<br>(60.6) | 72<br>(84.3) | 70<br>(85.6) | 27<br>(100) |

It is apparent from Table 4 that all the test compounds exhibited antimutagenic properties.

[Effects]

As described above in detail, all the selenium compounds used in the present invention have a remarkable life-prolonging effect on mice bearing cancer

and antimutagenic effect to bacteria. Thus, the use of them as antineoplastic agents is expected.

The following examples will show preparations of the antineoplastic agents of the present invention.

Preparation Example 1

One g of selenium oxychloride·quinoline (1:2) complex was dissolved in 200 ml of a physiological saline solution. The solution was filtered through a Millipore filter GS to remove microbes. Two ml of the filtrate was placed in a 10 ml ampoule and freeze-dried to obtain an injection containing 10 mg of the antineoplastic agent.

Preparation Example 2

Ten parts by weight of phenylseleninic acid, 120 parts of crystalline lactose, 167 parts of crystalline cellulose and 3 parts of magnesium stearate were mixed together in a V-type mixer and then shaped into tablets each containing 100 mg of the active ingredient with a tableting machine.

Claims:

1. An antineoplastic agent containing a selenium compound of the general formula:

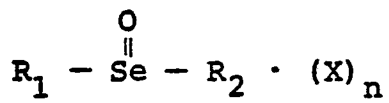

wherein $R_1$ represents a halogen atom, a phenyl group, a lower alkoxy group or a halogen-substituted lower alkoxy group, $R_2$ represents a halogen atom, a hydroxy group, a lower alkoxy group or a halogen-substituted lower alkoxy group or $R_1$ and $R_2$ together form a group of the formula:

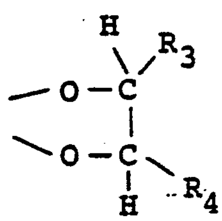

or

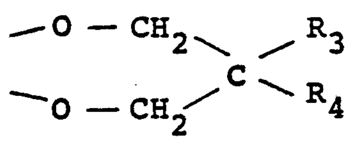

(in which $R_3$ and $R_4$ each represent a hydrogen atom or a lower alkyl group), X represents pyridine, quinoline, isoquinoline or dioxane and <u>n</u> represents an integer of 0 to 2,

or

$$Se(Hal)_4 \cdot (Y)_m$$

wherein Hal represents a halogen atom, Y represents pyridine and <u>m</u> represents 0 or 2.

2. An antineoplastic agent according to Claim 1 wherein $R_1$ and $R_2$ are each a chlorine atom or an ethoxy group or $R_1$ and $R_2$ together form a group:

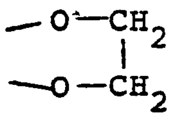

, n is 0 or 2 and X represents pyridine or quinoline.

3. An antineoplastic agent according to Claim 1 wherein Hal represents a chlorine or bromine atom and m represents 0 or 2.

4. An antineoplastic agent according to Claim 1 which contains a selenium oxychloride·pyridine (1:2) complex.

5. An antineoplastic agent according to Claim 1 which contains a selenium oxychloride·quinoline (1:2) complex.

6. An antineoplastic agent according to Claim 1 which contains diethyl selenite.

7. An antineoplastic agent according to Claim 1 which contains 1,2-ethanediol selenite.

8. An antineoplastic agent according to Claim 1 which contains selenium tetrachloride.

9. An antineoplastic agent according to Claim 1 which contains selenium tetrabromide.

10. An antineoplastic agent according to Claim 1 which contains a selenium tetrachloride·pyridine (1:2) complex.

11. A compound as defined in claim 1 for the treatment of tumors.

12. The use of a selenium compound of the following general formula for the preparation of antineoplastic agents:

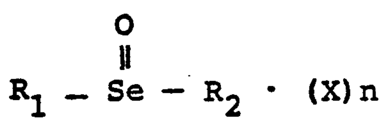

-- wherein $R_1$ represents a halogen atom, a phenyl group, a lower alkoxy group or a halogen-substituted lower alkoxy group, $R_2$ represents a halogen atom, a hydroxy group, a lower alkoxy group or a halogen-substituted lower alkoxy group or $R_1$ and $R_2$ together form a group of the formula:

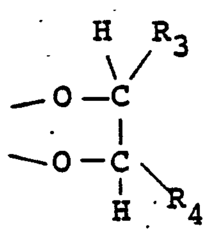

or

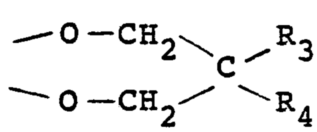

(in which $R_3$ and $R_4$ each represent a hydrogen atom or a lower alkyl group), X represents pyridine, quinoline, isoquinoline or dioxane and n represents

an integer of 0 to 2,

or

$$Se(Hal)_4 \cdot (Y)m$$

wherein Hal represents a halogen atom, Y represents pyridine and m represents 0 or 2.